# EUROPÄISCHE PATENTSCHRIFT

(11) **EP 1 652 549 B1**
(45) Veröffentlichungstag und Bekanntmachung des Hinweises auf die Patenterteilung: **20.01.2010**
(21) Anmeldenummer: 05020539.2
(22) Anmeldetag: 21.09.2005
(51) Int. Cl.: A61M 27/00

(54) **Anordnung zum Sammeln und Messen von Flüssigkeiten**
A device for collecting and measuring of fluids
Dispositif pour collecter et mesurer des liquides

(30) Priorität: 01.11.2004 DE 102004053166
(43) Veröffentlichungstag der Anmeldung: 03.05.2006
(73) Patentinhaber: PRIMED HALBERSTADT MEDIZINTECHNIK GMBH, 38820 Halberstadt (DE)
(72) Erfinder: Tautenhahn, Jörg Dr. med., 39116 Magdeburg (DE); Eder, Frank, Dr. med., 38820 Halberstadt (DE); Lippert, Hans, Prof. Dr., 39175 Wahlitz (DE); Leibitzki, Harry, 38889 Blankenburg (DE)
(74) Vertreter: Bock, Gerhard

(56) Entgegenhaltungen:
- FR-A- 2 749 174
- GB-A- 606 248
- US-A- 5 636 643
- US-A1- 2002 150 720
- US-B1- 6 682 506

## Beschreibung

Die Erfindung betrifft ein Vakuum-assistiertes Drainagesystem.

Bei der Wunddrainage unterscheidet man eine direkte Drainage (Drainageschlauch direkt in der Wunde liegend) und eine Schwammvermittelte Drainage (Drainageschlauch liegt in einem Schwamm, der in der Wunde liegt).

Die direkte Wunddrainage ist seit vielen Jahrzehnten bekannt. Vorrichtungen zur Wunddrainage unter Verwendung von Schwämmen, die in die Wunde eingeführt werden, bauen auf diesen Stand der Technik auf und wurden vor mehr als 60 Jahren entwickelt.

So offenbart bspw. die Schrift DE 847 475 eine Vorrichtung zur Drainage von Wunden mittels weicher Tampons, wobei eine mit ihrem einen Ende in einen Tampon aus zweckmäßiger Weise feinporigem und weich elastischem Natur-, Gummi-, Schaumgummi-, Zellstoffschwamm, Gaze, Watte oder dergleichen eingearbeitete, innerhalb des Tampons offene Saugleitung (oder mehrere solcher Leitungen) vorgesehen ist (sind). Die Saugleitung kann dabei über ein Gabelstück in mehrere Äste, die in den Tampon führen, unterteilt sein. Zusätzlich können bei dieser Vorrichtung noch weitere Rohre (Spülleitungen) zur Zuführung von Spül-, Desinfektions- oder Heilflüssigkeit zur Wunde vorhanden sein.

Aus der Schrift DE 696 13 997 T2 ist eine tragbare Wundbehandlungsvorrichtung bekannt, die aus einem porösen, für Flüssigkeiten durchlässigen Polster zum Einführen in die Wunde, einem Verband zur Abdeckung der Wunde und zum Bereitstellen einer im wesentlichen luftdichten Abdichtung um die Wunde herum, eine das Polster mit einer Saugpumpe verbindende Drainageleitung und wenigstens einem Filter aufgebaut ist, sodass an der Wunde gesaugt werden kann, um Flüssigkeiten von dort abzuziehen, wobei ein Druck-Nachweisgerät vorgesehen ist, das den Druck im wesentlichen an der wunden Stelle überwacht.

US 2002/0150720 offenbart einen Wunddrainage mit einem porösen, für Flüssigkeiten durchlässigen Polster zum Einführen in eine Wunde mit eingearbeiteter, innerhalb Polsters gelegener offener Drainageleitung, die mit einer Saugpumpe verbindbar ist.

Der Nachteil aller bekannten, Schwamm-vermittelten Wunddrainagevorrichtungen ist, dass das für Flüssigkeiten durchlässige Polster (der Schwamm) im in die Wunde eingeführten Zustand durch Feststoffe, die in den Flüssigkeiten vorhanden sein können, zugesetzt und somit verstopft werden, was zu einem Totalausfall bzgl. ihrer Drainagewirkung führt.

Der Erfindung liegt die Aufgabe zugrunde, ein Vakuum-assistiertes Drainagesystem anzugeben, das ein Verstopfen des für Flüssigkeiten durchlässigen Polsters (des Schwammes) verhindert und somit eine Drainagewirkung gewährleistet, auch wenn die abzuführenden Flüssigkeiten einen Feststoffanteil aufweisen.

Gemäß der Erfindung wird diese Aufgabe durch die kennzeichnenden Merkmale des ersten Patentanspruchs gelöst und durch vorteilhafte Ausgestaltungen gemäß den Unteransprüchen ergänzt.

Das Wesen der Erfindung besteht darin, dass das Vakuum-assistiertes Drainagesystem aus einem porösen, für Flüssigkeiten durchlässigen Polster zum Einführen in die Wunde, einem Verband zur Abdeckung des Polsters und der Wunde sowie zum Bereitstellen einer im Wesentlichen luftdichten Abdichtung um die Wunde herum und eine das Polster mit einer Saugpumpe verbindende Drainageleitung aufgebaut ist, sodass an der Wunde gesaugt werden kann, um Flüssigkeiten von dort abzuziehen, wobei das Polster mit einer durchgehenden Ausnehmung versehen ist, in dem ein starrer, röhrenförmiger, in die Ausnehmung einführbarer Abstandhalter platziert ist und ein Unterdruck unter dem Verband erzeugbar ist.
Über die Ausnehmung ist eine weitere Drainageleitung in die Wunde einführbar, um festere Bestandteile aus der Wunde zu entfernen.
Um während der Verwendung des erfindungsgemäßen Vakuum-assistierten Drainagesystems eine mögliche Gefäßverletzung anzuzeigen, bspw. während der Nachtstunden, ist dieses Drainagesystem mit einem Blutdetektor, bspw. Spektroskop mit optischer Faser, die mit ihrem Detektionsende in die Drainageleitung mündet, versehbar.

Die Erfindung wird nachstehend an Hand der schematischen Zeichnungen näher erläutert. Es zeigt:
- Fig. 1: eine Ausführungsform des erfindungsgemäßen Vakuumassistiertes Drainagesystems in einer schematischen Draufsicht
- Fig. 2: die Ausführungsform gem. Fig. 1 in einer schematischen Seitenansicht

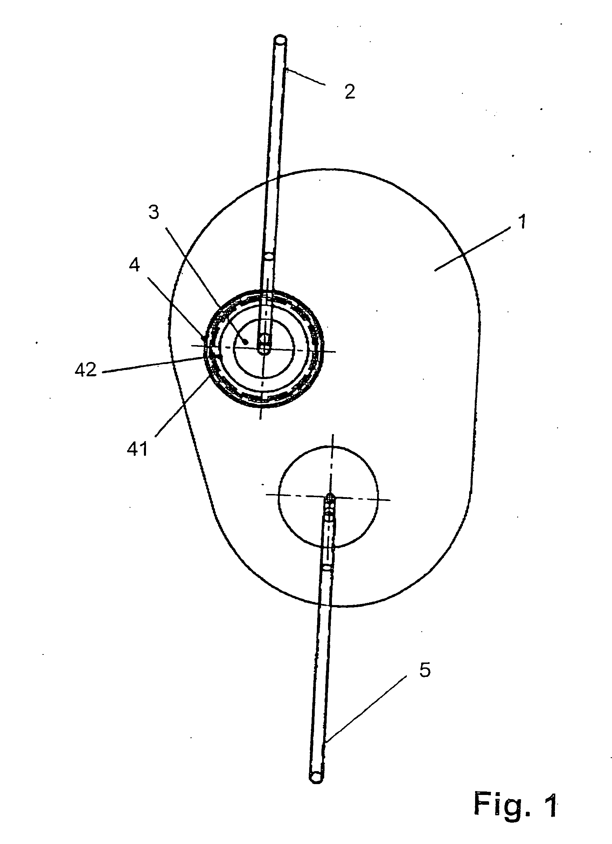

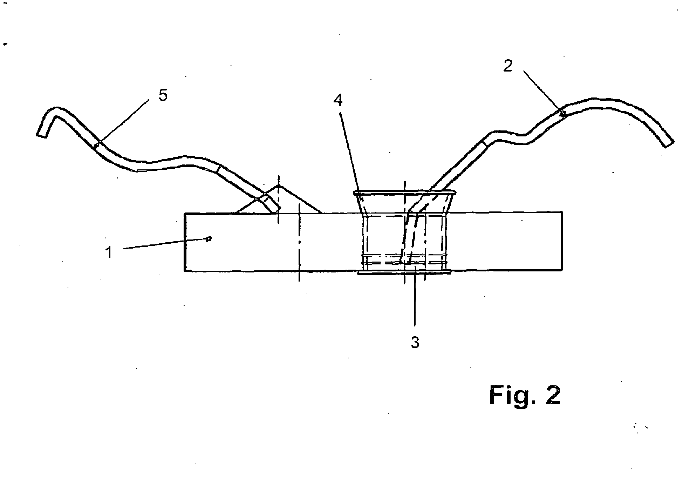

Das in Fig. 1 und 2 dargestellte Vakuum-assistiertes Drainagesystems besteht aus einem porösen, für Flüssigkeiten durchlässigen Polster (1) zum Einführen in eine Wunde, einem in der Figur aus Übersichtsgründen nicht dargestellten Verband zur Abdeckung des Polsters und der Wunde und zum Bereitstellen einer im wesentlichen luftdichten Abdichtung um die Wunde herum, eine das Polster (1) mit einer Saugpumpe verbindende Drainageleitung (2) aufgebaut ist, sodass an der Wunde gesaugt werden kann, um Flüssigkeiten von dort abzuziehen, wobei das Polster (1) mit einer durchgehenden Ausnehmung (3) versehen ist, in dem ein starrer, röhrenförmiger, in die Ausnehmung (3) einführbarer Abstandhalter (4) platziert ist.

Durch diesen Aufbau wird ein erster Drainageweg für hochflüssige und flüssige Körpersekrete, wie bspw. Lymphe, Blut oder Urin, aus der Wunde heraus ermöglicht.

Durch die Öffnung des Abstandhalters (4) hindurch ist eine zweite Drainageleitung (5) durchführbar.
Durch diese Drainageleitung (5) wird bei Bedarf ein zweiter Drainageweg für festflüssige Körpersekrete, wie bspw. Kot oder Eiter, aus der Wunde heraus ermöglicht.

Besonders vorteilhaft ist dieser Abstandhalter (4) aus Kunststoff und weist einen Innendurchmesser in einem Bereich von 1 bis 5 cm auf, der an die Verwendung anpasst in diesem Bereich frei wählbar ist. Diese Wählbarkeit kann bspw. durch verschiedene, in die Ausnehmung (3) einführbare Abstandhalter (4) unterschiedlicher Durchmesser erfolgen. Alternativ dazu besteht die Möglichkeit, über formschlüssig und dichtend in den Abstandhalter (4) einführbare, ebenfalls formschlüssig und dichtend ineinanderfügbare Abstandhalter (41), (42) den Innendurchmesser vorzugeben.

Das Polster (1) besteht besonders vorteilhaft aus einem verfestigten Kunststoffschaum mit einer Porengröße von 0,3 bis 2,5 mm.
Dieser Schaum kann bspw. ein Polyurethan-Schaum mit einer Porengröße von 0,35 bis 2,3 mm oder ein Polyvinylalkohol-Schaum mit einer Porengröße von 0,4 bis 2 mm sein.

Besonders vorteilhaft ist die Drainageleitung (2) an ihrer, dem Polster zugewandten Seite mit einem trichterförmigen Erweiterungsstück versehen (nicht in den Figuren dargestellt), dass einstückig als Ende der Drainageleitung (2) oder zweitstückig als ansteckbares Teil ausgeführt sein kann. Durch dieses Erweiterungsstück vergrößert sich der Querschnitt der auf dem Polster (1) aufliegenden Drainageleitung (2).

Der Verband (nicht in den Figuren dargestellt) ist besonders vorteilhaft mit einer abdeckenden Folienschicht versehen und an seinen Rändern mit einer Klebschicht versehen, so dass eine gute luftdichte Abdichtung gewährleistet wird.

Die Saugpumpe (nicht in den Figuren dargestellt) erzeugt vorteilhafter weise ein Vakuum von 50 mm Hg bis 375 mm Hg bei luftdichter Abdichtung der erfindungsgemäßen Vorrichtung um die Wunde herum.

Um während der Verwendung des erfindungsgemäßen Vakuum-assistierten Drainagesystems eine mögliche Gefäßverletzung anzuzeigen, bspw. während der Nachtstunden, ist dieses Drainagesystem mit einem Blutdetektor, bspw. Spektroskop mit optischer Faser, die mit ihrem Detektionsende in die Drainageleitung (2) mündet (aus Übersichtlichkeitsgründen nicht in den Figuren dargestellt), versehbar.

Durch die Erfindung wird ein Verstopfen des für Flüssigkeiten durchlässigen Polsters (1), bspw. durch Feststoffe wie Kot, verhindert, so dass eine Drainagewirkung auch gewährleistet ist, wenn die abzuführenden Flüssigkeiten einen Feststoffanteil aufweisen.

Durch den Blutdetektor ist es möglich, während des Saugvorgangs vermittels des erfindungsgemäßen Vakuum-assistierten Drainagesystems Gefäßverletzungen im Wundbereich festzustellen.

Das erfindungsgemäße Vakuum-assistierte Drainagesystem kommt bspw. bei der okklusiven Wundbehandlung im Abdominalbereich zum oder bei der Fistelseparation zum Einsatz.
Bei der okklusiven Wundbehandlung im Abdominalbereich wird das Polster (1) bspw. auf die Darmschlingen aufgelegt, wobei ein alloplastisches Netz oder eine Gaze zwischen dem Polster (1) und den Darmschlingen platziert, um einer Fistelbildung vorzubeugen.
Bei der Fistelseparation wird das Polster (1) über der Fistel positioniert und das Sekret um diese herum über das Polster (1) und die Drainageleitung(2) die abgesaugt, wobei die Drainageleitung (5) direkt an die Fistel herangeführt wird, um direkt über dieser abzusaugen.

Je nach Wundsituation empfiehlt es sich, das erfindungsgemäße Vakuum-assistierte Drainagesystem im Rahmen des Verbandwechsels alle 3 bis 5 Tage mit einem neuen Polster (1) zu versehen.

Der Einsatz des erfindungsgemäßen Vakuum-assistierten Drainagesystems führt bspw. zum Verschluss von komplizierten Abdominalwunden und bei rechtzeitiger Verwendung auch bei retrahierten, aber noch nicht mit dem Intestinium seitlich entzündlich verklebten Bauchdecken zu einem kompletten Bauchdeckenverschluss.

Das erfindungsgemäße Vakuum-assistierte Drainagesystem zeichnet sich durch hohen Pflege- und Patientenkomfort aus.
Alle in der Beschreibung, den nachfolgenden Ansprüchen und der Zeichnung dargestellten Merkmale können sowohl einzeln als auch in beliebiger Kombination miteinander erfindungswesentlich sein.

### Bezugszeichenliste

- 1 -: Polster
- 2 -: Drainageleitung
- 3 -: Ausnehmung
- 4 -: Abstandhalter
- 41 und 42 -: ineinanderfügbare Abstandhalter
- 5 -: zweite Drainageleitung

## Patentansprüche

1. Vakuum-assistiertes Drainagesystems bestehend aus einem porösen, für Flüssigkeiten durchlässigen Polster (1) zum Einführen in eine Wunde mit eingearbeiteter, innerhalb Polsters (1) gelegener offener Drainageleitung (2), die mit einer Saugpumpe verbindbar ist, sodass an der Wunde gesaugt werden kann, um Flüssigkeiten von dort abzuziehen, wobei ein Verband zur Abdeckung des Polsters (1) und der Wunde sowie zum Bereitstellen einer im Wesentlichen luftdichten Abdichtung um die Wunde herum vorgesehen ist, das Polster (1) mit einer durchgehenden Ausnehmung (3) versehen ist, in dem ein starrer, röhrenförmiger, in die Ausnehmung (3) einführbarer Abstandhalter (4) platziert ist und eine zweite Drainageleitung (5), die ebenfalls mit einer Saugpumpe verbindbar ist, durch die Öffnung des Abstandhalters (4) hindurch führbar ist, so dass ein Absaugen größerer, einen Verschluss des Polsters (1) verursachender Partikel aus der Wunde ermöglicht wird, **dadurch gekennzeichnet, dass** der in die Ausnehmung (3) einführbare Abstandhalter (4) einen variabelen Innendurchmesser aufweist, in dem dieser über formschlüssig und dichtend in den Abstandhalter (4) einführbare, ebenfalls formschlüssig und dichtend ineinanderfügbare Abstandhalter (41), (42) vorgebbar ist.

2. Vakuum-assistiertes Drainagesystems nach Patentanspruch 1, **dadurch gekennzeichnet, dass** Abstandhalter (4) einen Innendurchmesser von 1 bis 5 cm besitzt.

3. Vakuum-assistiertes Drainagesystems nach Patentanspruch 1, **dadurch gekennzeichnet, dass** der Abstandhalter (4) aus Kunststoff besteht.

4. Vakuum-assistiertes Drainagesystems nach Patentanspruch 1, **dadurch gekennzeichnet, dass** das Polster (1) aus einem verfestigten Kunststoffschaum mit einer Porengröße von 0,3 bis 2,5 mm besteht.

5. Vakuum-assistiertes Drainagesystems nach Patentanspruch 4, **dadurch gekennzeichnet, dass** der Schaum ein Polyurethan-Schaum mit einer Porengröße von 0,35 bis 2,3 mm ist.

6. Vakuum-assistiertes Drainagesystems nach Patentanspruch 4, **dadurch gekennzeichnet, dass** der Schaum ein Polyvinylalkohol-Schaum mit einer Porengröße von 0,4 bis 2 mm ist.

7. Vakuum-assistiertes Drainagesystems gemäß einem der voranstehenden Patentansprüche, **dadurch gekennzeichnet, dass** ein Blutdetektor in die Drainageleitung (2) mündet.

8. Vakuum-assistiertes Drainagesystems nach Patentanspruch 7, **dadurch gekennzeichnet, dass** der Blutdetektor aus einem Spektroskop mit optischer Faser, die mit ihrem Detektionsende in die Drainageleitung (2) mündet, besteht.

9. Vakuum-assistiertes Drainagesystems gemäß einem der voranstehenden Patentansprüche, **dadurch gekennzeichnet, dass** die Drainageleitung (2) an ihrer, dem Polster zugewandten Seite ein trichterförmiges Erweiterungsstück aufweist.

10. Vakuum-assistiertes Drainagesystems nach Patentanspruch 9, **dadurch gekennzeichnet, dass** das Erweiterungsstück einstückig als Ende der Drainageleitung (2) ausgeführt ist.

11. Vakuum-assistiertes Drainagesystems nach Patentanspruch 9, **dadurch gekennzeichnet, dass** das Erweiterungsstück zweitstückig als ansteckbares Teil ausgeführt ist.

12. Verfahren zum Betreiben des Vakuum-assistierten Drainagesystems nach einem oder mehreren der voranstehenden Ansprüche, bei dem ein Anschluss des Drainagesystems über die Drainageleitung (2) an eine Saugpumpe erfolgt und ein Vakuum von 50 mm Hg bis 375 mm Hg bei luftdichter Abdichtung des Drainagesystems an der Drainageleitung (2) angelegt wird, so dass durch die Drainageleitung (2) flüssige Bestandteile und durch die Drainageleitung (5) festere Bestandteile hindurchgesaugt werden.

## Claims

1. Vacuum-assisted drainage system consisting of a fluid-permeable porous pad (1) to be introduced into a wound with an integrated open drainage conduit (2) which is embedded within the pad (1) and can be connected to a suction pump thus allowing to suction at the wound in order to suck up liquids from it, and a dressing being included for covering the pad (1) and the wound as well as for ensuring a mainly air-tight sealing around the wound, the pad (1) being provided with a through opening (3) and a rigid, tube-type spacer (4) that can be inserted into the opening (3) and a second drainage conduit (5) that can also be connected to a suction pump and guided through the opening of the spacer (4) thus making it possible to suck up larger particles out of the wound that cause a closure of the pad (1), wherein the spacer (4), which can be inserted into the opening (3), has a variable inner diameter that can be predetermined by spacers (41), (42) introducible into the spacer (4) in a form-fit and sealing manner and said spacers (41), (42) can be interconnected in a form-fit and sealing manner, too.

2. Vacuum-assisted drainage system of claim 1, wherein the spacer (4) has an inner diameter from 1 to 5 cm.

3. Vacuum-assisted drainage system of claim 1, wherein the spacer (4) is made of plastic material.

4. Vacuum-assisted drainage system of claim 1, wherein the pad (1) is made of a reinforced plastic foam having a pore size from 0.3 to 2.5 mm.

5. Vacuum-assisted drainage system of claim 4, wherein the foam is polyurethane foam having a pore size from 0.35 to 2.3 mm.

6. Vacuum-assisted drainage system of claim 4, wherein the foam is polyvinyl alcohol foam having a pore size from 0.4 to 2 mm.

7. Vacuum-assisted drainage system of one of the previous claims, wherein a blood detector is lead into the drainage conduit (2).

8. Vacuum-assisted drainage system of claim 7, wherein the blood detector consists of a spectroscope with optical fibre and the detection end of said fibre ends in the drainage conduit (2).

9. Vacuum-assisted drainage system of one of the previous claims, wherein the pad-facing side of the drainage conduit (2) is provided with a funnel-shaped extension part.

10. Vacuum-assisted drainage system of claim 9, wherein the extension part is a one-part piece designed as the end of the drainage conduit (2).

11. Vacuum-assisted drainage system of claim 9, wherein the extension part is designed as an attachable two-part piece.

12. Method for using the vacuum-assisted drainage system of one or several of the previous claims in which the drainage system is connected via the drainage conduit (2) to a suction pump and a vacuum ranging from 50 mm Hg to 375 mm Hg is applied to the drainage conduit (2) with the drainage system being air-tight sealed so that fluid particles can be sucked off through the drainage conduit (2) and more solid particles can be extracted by suction through the drainage conduit (5).

## Revendications

1. Système de drainage à base d'un vide qui se compose d'un coussin (1) poreux, perméable aux liquides (1) à introduire dans une plaie, avec un drain (2) ouvert, incorporé et situé dans le coussin (1). Le drain peut être connecté à une pompe aspirante de sorte que l'aspiration à la plaie soit possible pour en enlever les liquides. Un bandage est prévu pour couvrir le coussin (1) et la plaie ainsi que pour fournir une obturation imperméable à l'air en premier lieu autour de la plaie. Le coussin (1) est prévu d'un creux (3) d'un seul tenant dans lequel on a positionné un écarteur (4) rigide, tubulaire qui peut être introduit dans le creux (3) ainsi que d'un deuxième drain (5) qui peut également être connecté à une pompe aspirante et qui peut être introduit par l'orifice de l'écarteur (4) de sorte que des particules plus grandes, qui mènent à l'obturation du coussin (1) puissent être aspirées de la plaie. Ce système est **caractérisé en ce que** l'écarteur (4) qui peut être introduit dans le creux (3) se distingue par un diamètre intérieur variable qui peut être fixé par des écarteurs (41), (42) également serrants et de forme complémentaire à insérer dans l'écarteur (4) serrants et de forme complémentaire.

2. Le système de drainage à base d'un vide suivant la revendication 1 est **caractérisé en ce que** l'écarteur (4) possède un diamètre intérieur de 1 à 5 cm.

3. Le système de drainage à base d'un vide suivant la revendication 1 est **caractérisé en ce que** l'écarteur (4) consiste en matière plastique.

4. Le système de drainage à base d'un vide suivant la revendication 1 est **caractérisé en ce que** le coussin (1) se compose d'une mousse solidifiée de matière plastique ayant une grosseur de pore de 0,3 à 2,5 mm.

5. Le système de drainage à base d'un vide suivant la revendication 4 est **caractérisé en ce que** la mousse est une mousse de polyuréthane ayant une grosseur de pore de 0,35 à 2,3 mm.

6. Le système de drainage à base d'un vide suivant la revendication 4 est **caractérisé en ce que** la mousse est une mousse d'alcool polyvinylique ayant une grosseur de pore de 0,4 à 2 mm.

7. Le système de drainage à base d'un vide suivant une des revendications dénommées ci-dessus est **caractérisé en ce qu'**un détecteur de sang se jette dans le drain (2).

8. Le système de drainage à base d'un vide suivant la revendication 7 est **caractérisé en ce qu'**un détecteur de sang se compose d'un spectroscope avec fibre optique dont la pointe de détection se jette dans le drain (2).

9. Le système de drainage à base d'un vide suivant une des revendications dénommées ci-dessus est **caractérisé en ce que** le drain (2) est muni d'une pièce d'extension en entonnoir sur son côté tourné vers le coussin.

10. Le système de drainage à base d'un vide suivant la revendication 9 est **caractérisé en ce que** la pièce d'extension est réalisée en une seule pièce comme bout du drain (2).

11. Le système de drainage à base d'un vide suivant la revendication 9 est **caractérisé en ce que** la pièce d'extension est réalisée en deux pièces comme pièce à emboîtement.

12. Le procédé pour le service du système de drainage à base d'un vide suivant une ou plusieures des revendications précédentes pour lequel le système de drainage est raccordé à une pompe aspirante par le drain (2) et un vide de 50 mm à 375 mm de Hg est appliqué lors d'une obturation imperméable à l'air du système de drainage au drain (2) de sorte que des composants liquides puissent être aspirés par le drain (2) et des composants plus solide puissent être aspirés par le drain (5).
